# EUROPEAN PATENT SPECIFICATION

(11) **EP 3 242 933 B1**
(45) Date of publication and mention of the grant of the patent: **27.02.2019**
(21) Application number: 16700404.3
(22) Date of filing: 08.01.2016
(51) Int. Cl.: C12N 15/86, A61K 9/00, A61K 48/00, A61K 38/57, A61K 35/28, C12N 5/0775, A61K 35/12

(54) **GENETICALLY MODIFIED MESENCHYMAL STEM CELLS EXPRESSING ALPHA-1 ANTITRYPSIN (AAT)**
GENETISCH MODIFIZIERTE ALPHA-1-ANTITRYPSIN (AAT) EXPRIMIERENDE MESENCHYMALE STAMMZELLEN
CELLULES SOUCHES MÉSENCHYMATEUSES GÉNÉTIQUEMENT MODIFIÉES EXPRIMANT L'ALPHA-1-ANTITRYPSINE (AAT)

(30) Priority: 08.01.2015 EP 15150454; 28.10.2015 EP 15191934
(43) Date of publication of application: 15.11.2017
(73) Proprietor: Apceth GmbH & Co. KG, 81377 München (DE)
(72) Inventor: GÜNTHER, Christine, 81479 München (DE); GEIGER-SCHREDELSEKER, Sabine, 81371 München (DE); HERMANN, Felix, 81375 München (DE); HUSS, Ralf, 83666 Waakirchen (DE); FORSTER, Daria Larissa, 86150 Augbusrg (DE)
(74) Representative: Hertin und Partner Rechts- und Patentanwälte PartG mbB
(86) International application number: PCT/EP2016/050271
(87) International publication number: WO 2016/110565

(56) References cited:
- WO-A1-2013/096458
- HONG LI ET AL: "Adipose tissue-derived mesenchymal stem cell-based liver gene delivery", JOURNAL OF HEPATOLOGY, ELSEVIER, AMSTERDAM, NL, vol. 54, no. 5, 28 July 2010 (2010-07-28), pages 930-938, XP028192152, ISSN: 0168-8278, DOI: 10.1016/J.JHEP.2010.07.051 [retrieved on 2010-11-03] cited in the application
- GHAEDI M ET AL: "Establishment of lentiviral-vector-mediated model of human alpha-1 antitrypsin delivery into hepatocyte-like cells differentiated from mesenchymal stem cells", TISSUE AND CELL, CHURCHILL LIVINGSTONE MEDICAL JOURNALS, EDINBURGH, GB, vol. 42, no. 3, 1 June 2010 (2010-06-01), pages 181-189, XP027069539, ISSN: 0040-8166, DOI: 10.1016/J.TICE.2010.03.007 [retrieved on 2010-06-04] cited in the application
- HONG LI ET AL: "Ex Vivo Transduction and Transplantation of Bone Marrow Cells for Liver Gene Delivery of [alpha]1-Antitrypsin", MOLECULAR THERAPY, vol. 18, no. 8, 1 August 2010 (2010-08-01) , pages 1553-1558, XP055254019, GB ISSN: 1525-0016, DOI: 10.1038/mt.2010.116 cited in the application
- SONG S ET AL: "Recombinant adeno-associated virus-mediated alpha-1 antitrypsin gene therapy prevents type I diabetes in NOD mice", GENE THERAPY, NATURE PUBLISHING GROUP, GB, vol. 11, no. 2, 1 January 2004 (2004-01-01), pages 181-186, XP002517682, ISSN: 0969-7128, DOI: 10.1038/SJ.GT.3302156
- SIYOUNG LEE ET AL: MOLECULAR MEDICINE, vol. 19, no. 1, 1 January 2013 (2013-01-01), page 1, XP055185328, ISSN: 1076-1551, DOI: 10.2119/molmed.2012.00308
- MARTIN S LORRAINE ET AL: "Safety and efficacy of recombinant alpha(1)-antitrypsin therapy in cystic fibrosis.", PEDIATRIC PULMONOLOGY FEB 2006, vol. 41, no. 2, February 2006 (2006-02), pages 177-183, XP002754890, ISSN: 8755-6863

## Description

The invention relates to genetically modified mesenchymal stem cell for use as a medicament in the treatment of medical conditions associated with inflammation and/or an unwanted immune response in subjects without an alpha1-antitrypsin (AAT) deficiency, wherein said stem cells comprise an exogenous nucleic acid comprising (i) an Alpha-1 antitrypsin (AAT) encoding region operably linked to (ii) a promoter or promoter/enhancer combination.

### BACKGROUND OF THE INVENTION

Mesenchymal stem cells (MSCs) are cells of non-haematopoietic origin that reside in the bone marrow and other tissues. MSCs are commonly considered to be multipotent adult progenitor cells that have the ability to differentiate into a limited number of cell lineages, such as osteoblasts, chondrocytes, and adipocytes. Studies have been conducted on the use of MSCs as a therapeutic entity based on this capacity to differentiate directly into these end-stage phenotypes, including the use of MSCs to promote or augment bone repair and for the repair of cartilage defects (Vilquin and Rosset, Regenerative Medicine 2006: 1, 4, p 589, and Veronesi et al, Stem Cells and Development 2013; 22, p 181). The isolation and cultivation of MSCs for a number of therapeutic indications has been described and represents a promising approach towards treating inflammation-associated disorders (for example WO 2010/119039).

MSCs are known to exhibit immune evasive properties after administration to a patient. MSCs have been shown to exhibit a beneficial immune modulatory effect in cases of transplantation of allogeneic donor material (Le Blanc et al, Lancet 2004: 363, p 1439), thereby reducing a potentially pathogenic alloreactivity and rejection. Furthermore, MSCs are known to exhibit anti-tumorigenic effects, for example against Kaposi's sarcoma (Khakoo et al, J Exp Med 2006: 203, p 1235). MSC treatment can also play a therapeutic role in wound healing. The therapeutic delivery of MSCs can be performed via systemic injection, followed by MSC homing to and engraftment within sites of injury (Kidd et al, Stem Cells 2009: 27, p 2614). Although it is clear that MSCs have a regenerative effect on injured tissue, their use as a delivery vehicle for therapeutic transgenic proteins of interest has not yet been fully explored.

Inflammatory disease and diseases associated with an unwanted immune response represent a significant cause of health problems and mortality worldwide. For example, inflammatory disease such as lung disease or autoimmune diseases represent medical conditions that require improved modulation of inflammation in order to provide effective treatment.

Diseases of the lung (such as respiratory disease) may seriously impact the well-being of patients and encompass a number of pathological conditions affecting the organs and tissues of the lung. Respiratory disease, as one example of a lung disease, includes conditions of the upper respiratory tract, trachea, bronchi, bronchioles, alveoli, pleura and pleural cavity, and the nerves and muscles of breathing. Respiratory diseases are among the leading causes of death worldwide. Lung infections (such as pneumonia and tuberculosis), lung cancer and chronic obstructive pulmonary disease (COPD) together accounted for 9.5 million deaths worldwide during 2008, one-sixth of the global total. According to the World Health Organization four respiratory disease categories appear in the global top 10 causes of mortality, together accounting for one in six deaths as well as one in 10 disability-adjusted life-years lost. In the 28 countries of the European Union, these diseases account for one in eight deaths. Treatment is typically directed to the use of anti-inflammatories, such as corticosteroids, in order to reduce inflammation. In light of the prevalence of lung diseases, novel strategies for their treatment are required.

Autoimmune diseases are medical conditions associated with an immune response of an individual against cells, substances or tissues of their own body. A substantial minority of the general population suffers from autoimmune diseases, which may be acute or chronic, and are often debilitating over long periods of time. In both autoimmune and inflammatory diseases, the medical condition typically arises through reactions of the human adaptive and/or innate immune systems, for example in autoimmune diseases against the body's own tissues or proteins.

One example of an autoimmune condition of significant importance is diabetes mellitus type 1. Diabetes type 1 accounts for 5% to 10% of all diabetes cases. Globally approximately 80000 youths are diagnosed per year and it is estimated that in the US a total of 3 million individuals have Diabetes type 1 (Chiang et al., Diabetes Care 2014: 37, pp 2034-55). In patients suffering Diabetes Type-1, pancreatic beta-cells are destroyed by an autoimmune reaction involving beta-cell autoantigens, macrophages, dendritic cells, B lymphocytes and T-cell lymphocytes. Due to the deficiency in pancreatic beta-cells in these patients insulin production is reduced resulting in elevated levels of glucose in the blood and urine. Present approaches at treatment typically rely on modulating insulin levels without directly addressing underlying immune system pathology.

One example of an inflammatory disease of significant importance is arthritis, for example gout (gouty arthritis). Gout is a medical condition characterized typically by recurrent acute inflammatory arthritis in a particular location leading to a swollen and painful joint. The metatarsal-phalangeal joint at the base of the big toe is a commonly affected area. Gout is commonly accepted to be caused by elevated levels of uric acid in the blood, leading to crystallization of uric acid, and deposition of the crystals in joints, tendons, and surrounding tissues in the body of a patient. Treatment is typically directed to the use of anti-inflammatories, such as nonsteroidal anti-inflammatory drugs (NSAIDs) or corticosteroids, or medications that block uric acid production.

A number of anti-inflammatory agents are known, such as steroidal and non-steroidal agents, which are used in treating conditions such as arthritis. However, many of these agents often lead to unwanted side effects, especially after prolonged use. The need for further anti-inflammatory agents is evident, in particular in the context of treating inflammatory or auto-immune diseases that show poor response to mild or short term treatments, and/or lead to unwanted side effects in patients who require long-term treatment.

Alpha-1 antitrypsin (also known as A1AT, AAT, PI, SERPINA1) is a ∼52kDa glycoprotein that is one of the most abundant endogenous serine protease inhibitors (SERPIN superfamily). AAT is considered to be an acute phase protein, whereby AAT concentration can increase many fold upon acute inflammation.

Alpha 1-antitrypsin deficiency (α1-antitrypsin deficiency, A1AD) is a medical disorder that causes defective production of alpha 1-antitrypsin (A1AT), leading to decreased A1AT activity in the blood and lungs, and deposition of excessive abnormal A1AT protein in liver cells. There are more than 75 known different mutations in SERPINA1, whereby 90% of the AATD cases are due to a PI*Z missense mutation: Glu342Lys. Deficiency of AAT leads to a chronic uninhibited tissue breakdown, whereby neutrophil elastase is free to break down alveolar interstitial elastin resulting in respiratory complications such as airway inflammation and emphysema.

The treatment of AAT-deficiency remains a challenge, although gene therapy approaches for reconstituting functional AAT have been proposed. For example, US2014/0142161A describes gene therapeutic methods for a treatment of AAT-deficiencies by using recombinant adeno-associated virus (rAAV) based vectors to augment the expression of AAT.

Li et al. (J Hepatol 2011: 54, pp 930-8), Ghaedi et al. (Tissue and Cell 2010: 42, pp 181-9) and Li et al. (Mol Ther 2010: 18, pp 1553-8) describe genetically modified mesenchymal stem cells that express AAT and discuss their potential use for a treatment of AAT-deficiencies, in particular in relation to the treatment of diseases of the liver. In light of the severity of disease caused by A1AD, novel strategies are required for augmenting AAT production in diseased subjects.

Despite recent preliminary advances in treating AAT-deficiency, the therapeutic potential of AAT in cell therapeutic approaches in subjects that are not inflicted by an AAT deficiency remains largely unexplored. Ghaedi et al. (J Gene Med 2011: 13, pp 171-80) demonstrated cytotoxic effects of mesenchymal stem cells expressing AAT on human umbilical cord vein endothelial cells (HUVEC) and discuss their possible use as angiogenic inhibitors. The application of cellular therapy based on the expression of transgenic AAT via MSCs in the treatment of inflammatory or autoimmune diseases has, to the knowledge of the inventors, not been previously suggested.

### SUMMARY OF THE INVENTION

In light of the prior art the technical problem underlying the present invention is to provide alternative and/or improved means for the treatment of inflammatory disease and diseases associated with an unwanted immune response.

This problem is solved by the features of the independent claims. Preferred embodiments of the present invention are provided by the dependent claims.

The invention therefore relates to a genetically modified mesenchymal stem cell for use as a medicament in the treatment of medical conditions associated with inflammation and/or an unwanted immune response in subjects without an alpha1-antitrypsin (AAT) deficiency, wherein said stem cells comprise an exogenous nucleic acid comprising (i) an Alpha-1 antitrypsin (AAT) encoding region operably linked to (ii) a promoter or promoter/enhancer combination. Such MSCs may be referred to as "AAT-modified MSCs".

In a preferred embodiment the invention relates to a genetically modified mesenchymal stem cell as described herein (AAT-modified MSC) for use as a medicament in the treatment of medical conditions associated with inflammation and/or an unwanted immune response in subjects without an alpha1-antitrypsin (AAT) deficiency, wherein the medical condition is selected from the group consisting of an inflammatory disease of the lung, gout, chronic fibrosis, autoimmune disease, in particular diabetes Type 1.

The various medical indications to be treated with the AAT-modified MSCs according to the present invention (in particular inflammatory disease of the lung, gout, chronic fibrosis, autoimmune disease, in particular diabetes Type 1) are unified by a common feature of inflammation and/or an unwanted immune response in subjects without an alpha1-antitrypsin (AAT) deficiency. Considering that the use of AAT-modified MSCs has not been previously proposed in the treatment of medical disorders not associated with alpha1-antitrypsin (AAT) deficiency, the various uses of the present invention represent a unified group of medical indications.

It was particularly surprising that AAT-modified MSCs are capable of treating medical conditions associated with an unwanted inflammation and/or an immune response in subjects that do not exhibit an AAT deficiency.

AAT replenishment in subjects with AAT deficiency has been proposed and appears to represent a useful approach towards treating a number of pathological conditions caused by reduced levels of functional AAT. However, it was not previously proposed that, in patients with functional AAT, increased local delivery of AAT to sites of inflammation via MSC-mediated delivery could provide a therapeutic solution to unwanted inflammation or immune responses. Instead, a person skilled in the art may have assumed that by administering MSC-AATs to subjects without an AAT-deficiency may lead to potential side effects due to an excess of AAT. Furthermore, the beneficial effects achieved by the invention would not be considered obvious in light of AAT replacement therapy in subjects with AAT deficiency, as the provision of excess AAT would not, as such, have been assumed to provide therapeutic benefit in patients with functional AAT.

According to the present invention the term Alpha 1-antitrypsin deficiency (α1-antitrypsin deficiency, A1AD) refers to any condition that causes defective production of alpha 1-antitrypsin (A1AT), such as those genetic disorders that lead to decreased A1AT activity in the blood and lungs, and deposition of excessive abnormal A1AT protein in liver cells. For example, an AAT deficiency may be characterized by one or more of the 75 known different mutations in SERPINA1, whereby 90% of the AATD cases are due to a PI*Z missense mutation: Glu342Lys.

Subjects without such AAT deficiency are therefore the preferred intended subjects for the medical treatments described herein.

Due to their ability to migrate to areas of inflammation, MSCs represent a suitable tool for the delivery of therapeutic agents. Without being bound by theory, the MSCs of the present invention represent a drug delivery vehicle for effective delivery of a therapeutic agent, namely AAT protein, expressed from an exogenous nucleic acid in said MSCs. Furthermore, the combination of MSCs with AAT provides unexpected synergistic effects. The MSCs as such exhibit beneficial properties with respect to anti-inflammatory effects in areas of inflammation after homing to and/or engraftment within inflamed tissue, and when coupled with AAT as the therapeutic transgene, provide an enhanced anti-inflammatory function.

Surprisingly, the administration of the MSCs described herein leads to migration of the MSCs to the sites of inflammation after systemic, preferably intravenous, administration of the cells. The MSCs are capable of migration and/or engraftment in areas of inflamed tissue, thereby providing anti-inflammatory signals from the MSCs themselves, in addition to the enhanced local anti-inflammatory effect of AAT from expression of the transgene present in the AAT-modified MSCs. The MSCs employed in the present invention are also capable of circulation in the blood stream after systemic or intravenous administration and may engraft or be bound to inflamed areas within the body, thereby exerting their function in a local manner. The combination of MSCs showing increased AAT expression (due to the AAT transgene) in the treatment of a medical condition defined by unwanted inflammation and/or an unwanted immune response provides an unexpected synergy, whereby the MSC homing to areas of elevated inflammation, in addition to the anti-inflammatory properties of both the MSCs themselves and the AAT transgene, provides a synergistic therapeutic effect greater than the sum of each individual effect when considered in an isolated fashion.

For example, when AAT is administered systemically as a therapeutic protein, or is administered in gene therapy in a viral vector, only limited benefits are achieved due to the lack of targeting the inflamed region within the body and the potential appearance of unwanted side effects due to off-target effects. When MSCs are administered systemically some positive effect may be obtained from the MSCs themselves due to their localization in inflamed tissue, although the strength of the anti-inflammatory response induced by unmodified MSCs may be insufficient to show a significant therapeutic effect. The combination of MSCs expressing AAT as a transgene, either under a constitutive or conditional promoter, enables a synergistic combination of the inherent properties of AAT and MSCs in a targeted and effective manner. For example, the local effects of AAT are beneficial in treating various inflammatory diseases, such as those characterized by an unwanted immune reaction. AAT is capable of protecting tissues from enzymes of inflammatory cells and can reduce the production of inflammatory cytokines.

Providing an excess of AAT via localized delivery by means of MSCs provides the desired therapeutic benefit in patients without AAT deficiency, while minimizing potential side effects of excess AAT due to off-target effects. In this process the immunomodulating properties of MSCs function synergistically with the anti-inflammatory effects of AAT to yield a therapeutically effective dose of AAT. The AAT-modified MSCs thereby avoid and/or minimize potential side effects due to systemic administration of AAT protein or AAT-encoding nucleic acid vectors. The use of MSCs as vehicles for AAT administration provides local production of AAT in diseased regions of the body due to the homing capabilities of MSCs towards inflamed tissue.

The AAT encoding region is preferably any nucleic acid that encodes a naturally occurring or synthetic AAT protein sequence that exhibits AAT function, with reduced, the same, similar or increased activity compared to human AAT, or is functionally analogous to AAT. The amino acid sequence of AAT is available under accession number 1313184B from the NCBI database. Corresponding nucleic acid sequences that encode AAT may be provided by one skilled in the art of molecular biology or genetics. The use of sequence variants of AAT that exhibit functional analogy to the unmodified human form of AAT is encompassed by the present invention.

The SERPINA1 coding sequence (CDS) as published at http://www.ncbi.nlm.nih.gov/nuccore/NM_000295.4 is one preferred embodiment and comprises bases 262 to 1518 of the full sequence (SEQ ID NO 1):

In some embodiments of the invention the CDS is codon optimized to increase protein production. The coding sequence after codon optimization preferably reads as follows (SEQ ID NO 2):

The nucleotide sequence according to SEQ ID NO 1 and/or 2 encodes a human AAT protein of the amino acid sequence according to SEQ ID NO 3, which is preferred in the present invnetion:

The invention therefore encompasses a genetically modified MSC as described herein comprising a nucleic acid molecule selected from the group consisting of:
a) a nucleic acid molecule comprising a nucleotide sequence that encodes AAT protein, such as according to SEQ ID NO 3, preferably by a nucleotide sequence according to SEQ ID NO 1 or 2,
b) a nucleic acid molecule which is complementary to a nucleotide sequence in accordance with a);
c) a nucleic acid molecule comprising a nucleotide sequence having sufficient sequence identity to be functionally analogous/equivalent to a nucleotide sequence according to a) or b), comprising preferably a sequence identity to a nucleotide sequence according to a) or b) of at least 70%, 80%, preferably 90%, more preferably 95%;
d) a nucleic acid molecule which, as a consequence of the genetic code, is degenerated into a nucleotide sequence according to a) through c); and
e) a nucleic acid molecule according to a nucleotide sequence of a) through d) which is modified by deletions, additions, substitutions, translocations, inversions and/or insertions and is functionally analogous/equivalent to a nucleotide sequence according to a) through d).

The invention therefoire encompasses a genetically modified MSC as described herein comprising a nucleotide sequence encoding an amino acid sequence according to SEQ ID NO 3. The invention encompasses further sequence variants of SEQ ID NO 3, in particular those of at least 70% sequence identity to SEQ ID NO 3, preferably at least 80%, 85%, 90%, or at least 95% sequence identity to SEQ ID NO 3. Such sequence variants are preferably functionally analogous or equivalent to the human AA disclosed herein. Variations in the length of the protein are also encompassed by the invention, in cases where the functional equivalence of human AAT of SEQ ID NO 3 is maintained. Truncations or extensions in the length of the protein of, for example, up 50 amino acids, 40, 30, 20, or 10 amino acids may maintain AAT activity and are therefore encompassed in the present invention.

Functionally analogous sequences refer to the ability to encode a functional AAT gene product and to enable the same or similar functional effect as human AAT. AAT function may be determined by its ability to inhibit a variety of proteases in vitro, such as trypsin, or by its ability to inhibit neutrophil elastase (as described below). Appropriate assays for determining protease activity or for determining neutrophil elastase activity are known to a skilled person.

Protein modifications to the AAT protein, which may occur through substitutions in amino acid sequence, and nucleic acid sequences encoding such molecules, are also included within the scope of the invention. Substitutions as defined herein are modifications made to the amino acid sequence of the protein, whereby one or more amino acids are replaced with the same number of (different) amino acids, producing a protein which contains a different amino acid sequence than the primary protein. In some embodiments this amendment will not significantly alter the function of the protein. Like additions, substitutions may be natural or artificial. It is well known in the art that amino acid substitutions may be made without significantly altering the protein's function. This is particularly true when the modification relates to a "conservative" amino acid substitution, which is the substitution of one amino acid for another of similar properties. Such "conserved" amino acids can be natural or synthetic amino acids which because of size, charge, polarity and conformation can be substituted without significantly affecting the structure and function of the protein. Frequently, many amino acids may be substituted by conservative amino acids without deleteriously affecting the protein's function. In general, the non-polar amino acids Gly, Ala, Val, Ile and Leu; the non-polar aromatic amino acids Phe, Trp and Tyr; the neutral polar amino acids Ser, Thr, Cys, Gin, Asn and Met; the positively charged amino acids Lys, Arg and His; the negatively charged amino acids Asp and Glu, represent groups of conservative amino acids. This list is not exhaustive. For example, it is well known that Ala, Gly, Ser and sometimes Cys can substitute for each other even though they belong to different groups.

Methods for the genetic modification of MSCs are known to those skilled in the art. Examples of suitable methods for genetic modification of MSCs are disclosed in WO 2010/119039 and WO 2008/150368.

In one embodiment the genetically modified cells as described herein are characterized in that said cells are obtained from bone marrow, umbilical cord, adipose tissue, or amniotic fluid.

In one embodiment the genetically modified cells as described herein are characterized in that said cells are CD34 negative.

In one embodiment the genetically modified cells as described herein are characterized in that said cells are human cells.

In one embodiment the genetically modified mesenchymal stem cell as described herein is characterized in that the exogenous nucleic acid comprises a viral vector, for example in the form of a viral expression construct, more preferably a retroviral vector, in particular a gamma retroviral vector.

In one embodiment the genetically modified mesenchymal stem cell as described herein is characterized in that the exogenous nucleic acid is or comprises a non-viral expression construct.

In one embodiment the genetically modified mesenchymal stem cell as described herein is characterized in that said cells further comprise (iii) a selection marker gene operably linked to (iv) a constitutive promoter or promoter/enhancer combination.

In one embodiment the genetically modified mesenchymal stem cell as described herein is characterized in that the promoter or promoter/enhancer combination is a constitutive promoter.

In another embodiment the genetically modified mesenchymal stem cell as described herein is characterized in that the promoter or promoter/enhancer combination is the CMV or EF2 promoter.

In a preferred embodiment the genetically modified mesenchymal stem cell as described herein is characterized in that the constitutive promoter is the EFS promoter.

In a preferred embodiment the genetically modified mesenchymal stem cell as described herein is characterized in that the constitutive promoter is the PGK promoter.

In a preferred embodiment the genetically modified mesenchymal stem cell as described herein is characterized in that the constitutive promoter is the EF1alpha promoter.

In one embodiment the genetically modified mesenchymal stem cell as described herein is characterized in that said promoter or promoter/enhancer combination is an inducible or conditional promoter.

In one embodiment the genetically modified mesenchymal stem cell as described herein is characterized in that the promoter is inducible upon differentiation of said cell post-administration. In one embodiment the genetically modified mesenchymal stem cell as described herein is characterized in that the promoter is the Tie2 promoter.

In one embodiment the genetically modified mesenchymal stem cell as described herein is characterized in that the promoter is an inflammation-specific promoter, preferably wherein said promoter is induced by inflammatory mediators or cytokines and/or induced when the genetically modified mesenchymal stem cell comes into proximity with inflamed tissue.

The inducible forms of the promoter are designed to exhibit inflammation specific and/or localized expression of the AAT protein. In combination with the homing and/or migratory properties of the MSCs, a synergistic effect is achieved, so that very little AAT protein is expressed or produced in areas in the body of the subject distinct from the diseased tissue or organ.

In one embodiment the genetically modified mesenchymal stem cell as described herein is characterized in that the promoter is the RANTES promoter.

In one embodiment the genetically modified mesenchymal stem cell as described herein is characterized in that the promoter is the HSP70 promoter.

It was surprising, in light of the prior art, that the expression of the inducible promoters mentioned herein led to sufficient expression of the therapeutic protein AAT upon appropriate stimulus at the site of inflammation. The promoters provided herein show suitable inducible properties for quick and strong expression of AAT upon entering into proximity with inflamed tissue. Moreover the anti-inflammatory and immunomodulating effects of AAT on the tissues are particularly pronounced when the expression of AAT is controlled by the mentioned inducible promotors. This leads to a synergistic effect, whereby the treatment of patients with a medical condition associated with an unwanted inflammation and/or an immune response is particularly effective when administering genetically modified MSCs expressing AAT under the control of Tie2 and/or the RANTES.

In a further aspect the invention is directed to the AAT-modified cells as such, without limitation to a particular medical use. In this respect, the various structural features of the AAT-modified cells described herein, such as the transgene sequence, promoter, additional vector components as described below and in the examples, and combinations thereof, represent contributions to the existing art that have not been previously described.

In a further aspect the invention relates to the genetically modified mesenchymal stem cell as described herein for use as a medicament.

In one embodiment the genetically modified mesenchymal stem cell for use as a medicament as described herein is characterized in that said cells are administered by introducing a therapeutically effective number of cells into the bloodstream of a patient.

In a further aspect the invention relates to the genetically modified mesenchymal stem cell as described herein for use as a medicament in the treatment of a lung disease.

In one embodiment the genetically modified mesenchymal stem cell for use as a medicament as described herein is characterized in that the lung disease is an inflammatory disease of the lung.

In one embodiment the genetically modified mesenchymal stem cell for use as a medicament as described herein is characterized in that the lung disease is a respiratory disease.

In further embodiments the genetically modified mesenchymal stem cell for use as a medicament as described herein is characterized in that the lung disease is acute lung injury, chronic obstructive pulmonary disease including chronic bronchitis, emphysema, bronchiectasis and bronchiolitis, acute respiratory distress syndrome, asthma, sarcoidosis, hypersensitivity pneumonitis and/or pulmonary fibrosis.

In one embodiment the genetically modified mesenchymal stem cell for use as a medicament as described herein is characterized in that said cells are administered by introducing a therapeutically effective number of cells to the lung of a patient by inhalation, optionally in combination with introduction of said cells into the bloodstream of a patient.

A lung disease according to the present invention may relate, but is not limited to, one or more of Acute Bronchitis, Acute Respiratory Distress Syndrome (ARDS), Asbestosis, Asthma, Bronchiectasis, Bronchiolitis, Bronchiolitis Obliterans Organizing Pneumonia (BOOP), Bronchopulmonary Dysplasia, Byssinosis, Chronic Bronchitis, Coccidioidomycosis (Cocci), COPD, Cryptogenic Organizing Pneumonia (COP), Cystic Fibrosis, Emphysema, Hantavirus Pulmonary Syndrome, Histoplasmosis, Human Metapneumovirus, Hypersensitivity Pneumonitis, Influenza, Lymphangiomatosis, Mesothelioma, Middle Eastern Respiratory Syndrome, Nontuberculosis Mycobacterium, Pertussis, Pneumoconiosis (Black Lung Disease), Pneumonia, Primary Ciliary Dyskinesia, Primary Pulmonary Hypertension, Pulmonary Arterial Hypertension, Pulmonary Fibrosis, Pulmonary Vascular Disease, Respiratory Syncytial Virus, Sarcoidosis, Severe Acute Respiratory Syndrome, Silicosis, Sleep Apnea, Sudden Infant Death Syndrome, or Tuberculosis.

In preferred embodiments the lung disease is selected from an inflammatory or restrictive lung disease, a respiratory tract infection and/or a pulmonary vascular disease or condition.

Inflammatory lung disease is typically characterized by a high neutrophil count, e.g. asthma, cystic fibrosis, emphysema, chronic obstructive pulmonary disorder or acute respiratory distress syndrome. Restrictive lung diseases are a category of respiratory disease characterized by a loss of lung compliance, causing incomplete lung expansion and increased lung stiffness, such as in infants with respiratory distress syndrome.

Respiratory tract infections can affect any part of the respiratory system. They are traditionally divided into upper respiratory tract infections and lower respiratory tract infections. The most common upper respiratory tract infection is the common cold. However, infections of specific organs of the upper respiratory tract such as sinusitis, tonsillitis, otitis media, pharyngitis and laryngitis are also considered upper respiratory tract infections. The most common lower respiratory tract infection is pneumonia, an infection of the lungs which is usually caused by bacteria, particularly Streptococcus pneumoniae in Western countries. Worldwide, tuberculosis is an important cause of pneumonia. Other pathogens such as viruses and fungi can cause pneumonia for example severe acute respiratory syndrome and pneumocystis pneumonia. A pneumonia may develop complications such as a lung abscess, a round cavity in the lung caused by the infection, or may spread to the pleural cavity.

One aspect of the invention is directed towards the treatment of vascular disease using genetically modified AAT-modified MSCs as described herein. Pulmonary vascular diseases are conditions that affect the pulmonary circulation. Examples are pulmonary embolism, a blood clot that forms in a vein, breaks free, travels through the heart and lodges in the lungs (thromboembolism). Large pulmonary emboli are fatal, causing sudden death. A number of other substances can also embolise (travel through the blood stream) to the lungs but they are rare, such as fat embolism (particularly after bony injury), amniotic fluid embolism (with complications of labour and delivery) or air embolism (iatrogenic - caused by invasive medical procedures). The following lung diseases may also be treated according to the present invention: Pulmonary arterial hypertension, elevated pressure in the pulmonary arteries; pulmonary edema, leakage of fluid from capillaries of the lung into the alveoli (or air spaces); pulmonary hemorrhage, inflammation and damage to capillaries in the lung resulting in blood leaking into the alveoli.

The genetically modified mesenchymal stem cell as described herein can also be used as a medicament in the treatment of a medical condition associated with Alpha 1-antitrypsin deficiency (α1-antitrypsin deficiency, A1AD), in patients with or without an Alpha 1-antitrypsin deficiency.

Examples of medical conditions associated with Alpha 1-antitrypsin deficiency are Cirrhosis, COPD, Pneumothorax, Asthma, Wegener's granulomatosis, Pancreatitis, Gallstones, Bronchiectasis, Pelvic organ prolapse, Primary sclerosing cholangitis, Autoimmune hepatitis, Emphysema, predominantly involving the lower lobes and causing bullae and Secondary Membranoproliferative Glomerulonephritis.

In a further aspect the invention relates to the genetically modified mesenchymal stem cell as described herein for use as a medicament in the treatment of an inflammatory disorder.

In further embodiments the genetically modified mesenchymal stem cell for use as a medicament as described herein is characterized in that the inflammatory disease is vasculitis, nephritis, inflammatory bowel disease, rheumatoid arthritis and/or Graft versus Host disease.

In one embodiment the genetically modified mesenchymal stem cell as described herein for use as a medicament is characterized in that the inflammatory disease to be treated is gout. Gout is a medical condition characterized typically by recurrent acute inflammatory arthritis in a particular location leading to a swollen and painful joint. The metatarsal-phalangeal joint at the base of the big toe is a commonly affected area. Gout is commonly accepted to be caused by elevated levels of uric acid in the blood, leading to crystallization of uric acid, and deposition of the crystals in joints, tendons, and surrounding tissues in the body of a patient. Gout may in some cases appear as tophi, kidney stones, or urate nephropathy.

Surprisingly the AAT-modified MSCs of the present invention enable an effective therapeutic option for treating gout, leading to reductions in swelling and pain of an affected area. Successful therapy can be observed via reduced inflammation and reduced uric acid crystals in joint fluid. In a further aspect the invention relates to the genetically modified mesenchymal stem cell as described herein for use as a medicament in the treatment of chronic fibrosis.

In one embodiment the genetically modified mesenchymal stem cell for use as a medicament as described herein is characterized in that the inflammatory and/or chronic fibrotic disease is of the kidney, liver and/or colon of a subject.

Fibrosis is typically considered to be the formation of excess fibrous connective tissue in an organ or tissue. Fibrosis can be a reactive, benign, or pathological state. Unwanted deposition of connective tissue can obliterate the architecture and function of the underlying organ or tissue, thereby leading to a pathologic state. Fibrosis can occur in many tissues within the body, typically as a result of inflammation or damage potentially associated with an unwanted inflammation and/or immune response. Examples of fibrosis include fibrosis of the lungs (pulmonary fibrosis, idiopathic pulmonary fibrosis, cystic fibrosis), liver (Cirrhosis), heart (endomyocardial fibrosis, old myocardial infarction, atrial Fibrosis) or other causes (mediastinal fibrosis (soft tissue of the mediastinum), myelofibrosis (bone marrow), retroperitoneal fibrosis (soft tissue of the retroperitoneum), progressive massive fibrosis (lungs); a complication of coal workers' pneumoconiosis, mephrogenic systemic fibrosis (skin), crohn's Disease (intestine), keloid (skin), scleroderma/systemic sclerosis (skin, lungs), arthrofibrosis (knee, shoulder, other joints), Dupuytren's contracture (hands,fingers) or adhesive capsulitis (shoulder)).

It was surprising that the localized expression of AAT in fibrotic regions could lead to enhanced therapeutic effect. MSCs could show unexpected migratory properties towards fibrotic tissue and via expression of AAT lead to a reduction in the formation of fibrotic tissue. In this process AAT-MSC act in particular as an anti-inflammatory and/or immunomodulating therapeutic agent.

The treatment of type I diabetes using the AAT-modified MSCs described herein represents a further aspect of the present invention. The treatment of complications of type I diabetes is therefore also encompassed by the invention. Potential complications associated with Diabetes Type 1 include cardiovascular diseases in particular an accelerated progression of atherosclerosis as well as an elevated risk of a heart attack and/or stroke, nephropathy, neuropathy and retinopathy.

In one embodiment the genetically modified mesenchymal stem cell for use as a medicament as described herein is characterized in that the subject is human.

In one embodiment the genetically modified mesenchymal stem cell for use as a medicament as described herein is characterized in that said genetically modified cells are allogeneic with respect to the subject.

In one embodiment the genetically modified mesenchymal stem cell for use as a medicament as described herein is characterized in that said genetically modified cells are autologous with respect to the subject.

A further aspect of the invention relates to the AAT-modified MSCs as described herein for the treatment of inflammatory or autoimmune disease, such as those described herein, wherein cancer is excluded from the group of diseases to be treated.

In a further aspect of the invention either genetically modified or unmodified MSCs are provided for use as a medicament in the treatment of lung disease. The features of the cells as mentioned herein with respect to AAT-modified MSCs also apply to this further embodiment of the present invention. According to this embodiment the MSCs do not comprise an AAT-encoding nucleic acid. Either no genetic modification may be present, or the MSCs may comprise exogenous nucleic acids encoding therapeutic proteins, such as AAT. The invention therefore relates to the treatment of lung disease using MSCs, independent of whether modification of the MSCs has occurred or the particular genetic modification that has occurred. The anti-inflammatory properties of the MSCs lead to surprisingly good efficacy in the treatment of lung diseases as described herein. The potential lung diseases to be treated as mentioned herein with respect to treatment with AAT-modified MSCs are also applicable to this particular embodiment of the invention.

In a further aspect of the invention the MSC as described herein may comprise an exogenous nucleic acid encoding the protein CXCR4 in combination with further nucleic acid sequences suitable for expression of said protein. CXCR4 is a cell surface chemokine receptor that is involved in the mobilization of MSCs, and is expressed on the surface of a small proportion of MSCs. The expression of CXCR4 has been suggested to play a role in the efficiency of homing of MSCs towards tissue damage. Recent results have shown that the expression of CXCR4 in MSCs enhances the chemotactic and paracrine characteristics of the cells in vitro and improved MSC homing and colonization of damaged lung tissue in vivo. CXCR4-encoding sequences may be either present in the same exogenous nucleic acid molecule that encodes AAT or in a separate exogenous nucleic acid. Multiple integrated nucleic acid constructs or cassettes may be present in the MSCs of the present invention, each carrying one or more genes of interest, for example therapeutic genes, such as AAT or other genes involved in mobilization of the cells, such as CXCR4.

### DETAILED DESCRIPTION OF THE INVENTION

The "mesenchymal cells" disclosed herein (also referred to in some embodiments as "mesenchymal stem cells" or "MSCs") can give rise to connective tissue, bone, cartilage, and cells in the circulatory and lymphatic systems. Mesenchymal stem cells are found in the mesenchyme, the part of the embryonic mesoderm that consists of loosely packed, fusiform or stellate unspecialized cells. As used herein, mesenchymal stem cells include, without limitation, CD34-negative stem cells.

In one embodiment of the invention, the mesenchymal cells are fibroblast-like plastic adherent cells, defined in some embodiments as multipotent mesenchymal stromal cells and also include CD34-negative cells.

For the avoidance of any doubt, the term mesenchymal cell encompasses multipotent mesenchymal stromal cells that also includes a subpopulation of mesenchymal cells, MSCs and their precursors, which subpopulation is made up of multipotent or pluripotent self-renewing cells capable of differentiation into multiple cell types in vivo.

As used herein, CD34-negative cell shall mean a cell lacking CD34, or expressing only negligible levels of CD34, on its surface. CD34-negative cells, and methods for isolating such cells, are described, for example, in Lange C. et al., "Accelerated and safe expansion of human mesenchymal stromal cells in animal serum-free medium for transplantation and regenerative medicine". J. Cell Physiol. 2007, Apr. 25.

Mesenchymal cells can be differentiated from hematopoietic stem cells (HSCs) by a number of indicators. For example, HSCs are known to float in culture and to not adhere to plastic surfaces. In contrast, mesenchymal cells adhere to plastic surfaces. The CD34-negative mesenchymal cells of the present invention are adherent in culture.

The genetically modified cell(s) described herein may comprise different types of carriers depending on whether it is to be administered in solid, liquid or aerosol form, and whether it need to be sterile for such routes of administration as injection. The present invention can be administered intravenously, intradermally, intraarterially, intraperitoneally, intralesionally, intracranially, intraarticularly, intraprostatically, intrapleurally, intratracheally, intranasally, intravitreally, intravaginally, intrarectally, topically, intratumorally, intramuscularly, intraperitoneally, subcutaneously, subconjunctival, intravesicularlly, mucosally, intrapericardially, intraumbilically, intraocularally, orally, topically, locally, inhalation (e.g., aerosol inhalation), injection, infusion, continuous infusion, localized perfusion bathing target cells directly, via a catheter, via a lavage, in cremes, in lipid compositions (e.g., liposomes), or by other method or any combination of the forgoing as would be known to one of ordinary skill in the art (see, for example, Remington's Pharmaceutical Sciences, 18th Ed. Mack Printing Company, 1990).

The present invention encompasses treatment of a patient by introducing a therapeutically effective number of cells into a subject's bloodstream. As used herein, "introducing" cells "into the subject's bloodstream" shall include, without limitation, introducing such cells into one of the subject's veins or arteries via injection. Such administering can also be performed, for example, once, a plurality of times, and/or over one or more extended periods. A single injection is preferred, but repeated injections over time (e.g., quarterly, half-yearly or yearly) may be necessary in some instances. Such administering is also preferably performed using an admixture of CD34-negative cells and a pharmaceutically acceptable carrier. Pharmaceutically acceptable carriers are well known to those skilled in the art and include, but are not limited to, 0.01-0.1 M and preferably 0.05 M phosphate buffer or 0.8% saline, as well as commonly used proprietary cryopreservation media.

Administration may also occur locally, for example by injection into an area of the subject's body in proximity to a site of inflammation. MSCs have been shown to migrate towards inflammation. Mesenchymal stem cells (MSC) exhibit tropism for sites of tissue damage as well as the tumor microenvironment. Many of the same inflammatory mediators that are secreted by wounds are found in the tumor microenvironment and are thought to be involved in attracting MSC to these sites. Cell migration is dependent on a multitude of signals ranging from growth factors to chemokines secreted by injured cells and/or respondent immune cells. MSC are likely to have chemotactic properties similar to other immune cells that respond to injury and sites of inflammation. Regardless, the local administration of the cells as described herein may lead to high levels of the cells at their site of action.

Additionally, such pharmaceutically acceptable carriers can be aqueous or non-aqueous solutions, suspensions, and emulsions, most preferably aqueous solutions. Aqueous carriers include water, alcoholic/aqueous solutions, emulsions and suspensions, including saline and buffered media. Parenteral vehicles include sodium chloride solution, Ringer's dextrose, dextrose and sodium chloride, lactated Ringer's and fixed oils. Intravenous vehicles include fluid and nutrient replenishers, electrolyte replenishers such as Ringer's dextrose, those based on Ringer's dextrose, and the like. Fluids used commonly for i.v. administration are found, for example, in Remington: The Science and Practice of Pharmacy, 20th Ed., p. 808, Lippincott Williams S-Wilkins (2000). Preservatives and other additives may also be present, such as, for example, antimicrobials, antioxidants, chelating agents, inert gases, and the like.

As used herein, a "therapeutically effective number of cells" includes, without limitation, the following amounts and ranges of amounts: (i) from about 1 x 10² to about 1 x 10⁸ cells/kg body weight; (ii) from about 1 x 10³ to about 1 x 10⁷ cells/kg body weight; (iii) from about 1 x 10⁴ to about 1 x 10⁶ cells/kg body weight; (iv) from about 1 x 10⁴ to about 1 x 10⁵ cells/kg body weight; (v) from about 1 x 10⁵ to about 1 x 10⁶ cells/kg body weight; (vi) from about 5 x 10⁴ to about 0.5 x 10⁵ cells/kg body weight; (vii) about 1 x 10³ cells/kg body weight; (viii) about 1 x 10⁴ cells/kg body weight; (ix) about 5 x 10⁴ cells/kg body weight; (x) about 1 x 10⁵ cells/kg body weight; (xi) about 5 x 10⁵ cells/kg body weight; (xii) about 1 x 10⁶ cells/kg body weight; and (xiii) about 1 x 10⁷ cells/kg body weight. Human body weights envisioned include, without limitation, about 5 kg, 10 kg, 15 kg, 30 kg, 50 kg, about 60 kg; about 70 kg; about 80 kg, about 90 kg; about 100 kg, about 120 kg and about 150 kg. These numbers are based on pre-clinical animal experiments and human trials and standard protocols from the transplantation of CD34+ hematopoietic stem cells. Mononuclear cells (including CD34+ cells) usually contain between 1:23000 to 1:300000 CD34-negative cells.

As used herein, "treating" a subject afflicted with a disorder, such as inflammation, shall mean slowing, stopping or reversing the disorder's progression. In the preferred embodiment, treating a subject afflicted with a disorder means reversing the disorder's progression, ideally to the point of eliminating the disorder itself. As used herein, ameliorating a disorder and treating a disorder are equivalent. The treatment of the present invention may also, or alternatively, relate to a prophylactic administration of said cells. Such a prophylactic administration may relate to the prevention of any given medical disorder, such as the prevention of inflammation, or the prevention of development of said disorder, whereby prevention or prophylaxis is not to be construed narrowly under all conditions as absolute prevention. Prevention or prophylaxis may also relate to a reduction of the risk of a subject developing any given medical condition, preferably in a subject at risk of said condition.

Typically, the term "inflammation" as used in its art-recognized sense relates to a localized or systemic protective response elicited by injury, infection or destruction of tissues which serves to protect the subject from an injurious agent and the injured tissue. Inflammation is preferably characterized by fenestration of the microvasculature, leakage of the elements of blood into the interstitial spaces, and migration of leukocytes into the inflamed tissue, which may lead to an uncontrolled sequence of pain, heat, redness, swelling, and loss of function.

Inflammation can be classified as either acute or chronic. Acute inflammation is the initial response of the body to harmful stimuli and is achieved by the increased movement of plasma and leukocytes (especially granulocytes) from the blood into the injured tissues. A cascade of biochemical events propagates and matures the inflammatory response, involving the local vascular system, the immune system, and various cells within the injured tissue. Prolonged inflammation, known as chronic inflammation, leads to a progressive shift in the type of cells present at the site of inflammation and is characterized by simultaneous destruction and healing of the tissue from the inflammatory process.

The term "unwanted inflammation" preferably refers to an inflammation in a subject that exceeds the level of a physiological beneficial inflammatory reaction and leads to damages of the cells, tissues and/or organs at the site of the inflammation.

The term "unwanted immune response" preferably refers an alteration of the reactivity of the immune system in a subject that has deleterious effect on its health and may involve the stimulation and/or production of cytokines as well as the recruitment of immune cells. Unwanted immune responses occur for instance in autoimmune diseases, transplant rejections, allergies or inflammatory diseases.

Medical conditions that are defined by unwanted inflammation and/or an immune response refer therefore to a number of diseases and/or disorders including in particular but not being limited to vasculitis, nephritis, inflammatory bowel disease, rheumatoid arthritis, Graft versus Host disease, gouty arthritis, chronic fibrosis, inflammatory diseases of the lung or autoimmune diseases.

Examples of inflammatory diseases of the lung include but are not limited to lung injury, chronic obstructive pulmonary disease (COPD) including chronic bronchitis, emphysema, bronchiectasis and bronchiolitis, acute respiratory distress syndrome, asthma, sarcoidosis, hypersensitivity pneumonitis and/or pulmonary fibrosis. In some embodiments of the invention the MSCs as described herein migrate towards physiological niches affected by a disease condition, such as areas of inflammation, in order to impart their therapeutic effect, for example in a local manner.

The invention is further directed towards the treatment of autoimmune disorders, in particular those with an inflammatory component. These disorders may also be referred to as rheumatic disorders. Such conditions are preferably selected from Takayasu Arteritis, Giant-cell arteritis, familial Mediterranean fever, Kawasaki disease, Polyarteritis nodosa, cutanous Polyarteritis nodosa, Hepatitis-associated arteritis, Behcet's syndrome, Wegener's granulomatosis, Churg-Strauss syndrome, microscopic polyangiitis, Vasculitis of connective tissue diseases, Hennoch-Schonlein purpura, Cryoglobulinemic vasculitis, Cutaneous leukocytoclastic angiitis, Tropical aortitis, Sarcoidosis, Cogan's syndrome, Wiskott-Aldrich Syndrome, Lepromatous arteritis, Primary angiitis of the CNS, Thromboangiitis obliterans, Paraneoplastic ateritis, Urticaria, Dego's disease, Myelodysplastic syndrome, Eythema elevatum diutinum, Hyperimmunoglobulin D, Allergic Rhinitis, Asthma bronchiale, chronic obstructive pulmonary disease, periodontitis, Rheumatoid Arthritis, atherosclerosis, Amyloidosis, Morbus Chron, Colitis ulcerosa, Autoimmune Myositis, Diabetes mellitus, Multiple sclerosis, Guillain-Barre Syndrome, histiocytosis, Osteoarthritis, atopic dermatitis, periodontitis, chronic rhinosinusitis, Psoriasis, psoriatic arthritis, Microscopic colitis, Pulmonary fibrosis, glomerulonephritis, Whipple's disease, Still's disease, erythema nodosum, otitis, cryoglobulinemia, Sjogren's syndrome, Lupus erythematosus, aplastic anemia, Osteomyelofibrosis, chronic inflammatory demyelinating polyneuropathy, Kimura's disease, systemic sclerosis, chronic periaortitis, chronic prostatitis, idiopathic pulmonary fibrosis, chronic granulomatous disease, Idiopathic achalasia, bleomycin-induced lung inflammation, cytarabine-induced lung inflammation, Autoimmunthrombocytopenia, Autoimmunneutropenia, Autoimmunhemolytic anemia, Autoimmunlymphocytopenia, Chagas' disease, chronic autoimmune thyroiditis, autoimmune hepatitis, Hashimoto's Thyroiditis, atropic thyroiditis, Graves disase, Autoimmune polyglandular syndrome, Autoimmune Addison Syndrome, Pemphigus vulgaris, Pemphigus foliaceus, Dermatitis herpetiformis, Autoimmune alopecia, Vitiligo, Antiphospholipid syndrome, Myasthenia gravis, Stiff-man syndrome, Goodpasture's syndrome, Sympathetic ophthalmia, Folliculitis, Sharp syndrome and/or Evans syndrome.

As used herein "cell migration" is intended to mean movement of a cell towards a particular chemical or physical signal. Cells often migrate in response to specific external signals, including chemical signals and mechanical signals. Chemotaxis is one example of cell migration regarding response to a chemical stimulus. In vitro chemotaxis assays such as Boyden chamber assays may be used to determine whether cell migration occurs in any given cell. For example, the cells of interest may be purified and analysed. Chemotaxis assays (for example according to Falk et al., 1980 J. Immuno. Methods 33:239-247) can be performed using plates where a particular chemical signal is positioned with respect to the cells of interest and the transmigrated cells then collected and analyzed. For example, Boyden chamber assays entail the use of chambers isolated by filters, used as tools for accurate determination of chemotactic behavior. The pioneer type of these chambers was constructed by Boyden (Boyden (1962) "The chemotactic effect of mixtures of antibody and antigen on polymorphonuclear leucocytes". J Exp Med 115 (3): 453). The motile cells are placed into the upper chamber, while fluid containing the test substance is filled into the lower one. The size of the motile cells to be investigated determines the pore size of the filter; it is essential to choose a diameter which allows active transmigration. For modelling in vivo conditions, several protocols prefer coverage of filter with molecules of extracellular matrix (collagen, elastin etc.) Efficiency of the measurements can be increased by development of multiwell chambers (e.g. NeuroProbe), where 24, 96, 384 samples are evaluated in parallel. Advantage of this variant is that several parallels are assayed in identical conditions.

Alternatively, tissue samples may be obtained from subjects (for example rodent models) after cell transplantation and assayed for the presence of the cells of interest in particular tissue types. Such assays may be of molecular nature, identifying cells based on nucleic acid sequence, or of histological nature, assessing cells on the basis of fluorescent markings after antibody labeling. Such assays are also particularly useful for assessing engraftment of transplanted cells. Assays for engraftment may also provide information on cell migration, as to some extent the engraftment is dependent on cell localization prior to engraftment.

In some embodiments of the invention the MSCs as described herein engraft in physiological niches affected by a disease condition, such as areas of inflammation, in order to impart their therapeutic effect, for example in a local manner.

As used herein "engraftment" relates to the process of incorporation of grafted or transplanted tissue or cells into the body of the host. Engraftment may also relate to the integration of transplanted cells into host tissue and their survival and under some conditions differentiation into non-stem cell states.

Techniques for assessing engraftment, and thereby to some extent both migration and the biodistribution of MSCs, can encompass either in vivo or ex vivo methods. Examples of in vivo methods include bioluminescence, whereby cells are transduced to express luciferase and can then be imaged through their metabolism of luciferin resulting in light emission; fluorescence, whereby cells are either loaded with a fluorescent dye or transduced to express a fluorescent reporter which can then be imaged; radionuclide labeling, where cells are loaded with radionuclides and localized with scintigraphy, positron emission tomography (PET) or single photon emission computed tomography (SPECT); and magnetic resonance imaging (MRI), wherein cells loaded with paramagnetic compounds (e.g., iron oxide nanoparticles) are traced with an MRI scanner. Ex vivo methods to assess biodistribution include quantitative PCR, flow cytometry, and histological methods. Histological methods include tracking fluorescently labeled cells; in situ hybridization, for example, for Y-chromosomes and for human-specific ALU sequences; and histochemical staining for species-specific or genetically introduced proteins such as bacterial β-galactosidase. These immunohistochemical methods are useful for discerning engraftment location but necessitate the excision of tissue. For further review of these methods and their application see Kean et al., MSCs: Delivery Routes and Engraftment, Cell-Targeting Strategies, and Immune Modulation, Stem Cells International, Volume 2013 (2013).

Progenitor or multipotent cells, such as the mesenchymal cells of the present invention, may therefore be described as protein delivery vehicles, essentially enabling the localization and expression of therapeutic gene products in particular tissues or regions of the subject's body.

Such therapeutic cells offer the potential to provide cellular therapies for diseases that are refractory to other treatments. For each type of therapeutic cell the ultimate goal is the same: the cell should express a specific repertoire of genes, preferably exogenous nucleic acids that code for therapeutic gene products, thereby modifying cell identity to express said gene product and provide a therapeutic effect, such as an anti-inflammatory effect. The cells of the invention, when expanded in vitro, represent heterogeneous populations that include multiple generations of mesenchymal (stromal) cell progeny, which lack the expression of most differentiation markers like CD34. These populations may have retained a limited proliferation potential and responsiveness for terminal differentiation and maturation along mesenchymal and non-mesenchymal lineages.

As used herein "inducible expression" or "conditional expression" relates to a state, multiple states or system of gene expression, wherein the gene of interest, such as the therapeutic transgene, is preferably not expressed, or in some embodiments expressed at negligible or relatively low levels, unless there is the presence of one or more molecules (an inducer) or other set of conditions in the cell that allows for gene expression. Inducible promoters may relate to either naturally occurring promoters that are expressed at a relatively higher level under particular biological conditions, or to other synthetic promoters comprising any given inducible element. Inducible promoters may refer to those induced by particular tissue- or micro-environments or combinations of biological signals present in particular tissue- or micro-environments, or to promoters induced by external factors, for example by administration of a small drug molecule or other externally applied signal.

As used herein, in "proximity with" a tissue includes, for example, within 5 mm, within 1 mm of the tissue, within 0.5 mm of the tissue and within 0.25 mm of the tissue.

Given that stem cells can show a selective migration to different tissue microenvironments in normal as well as diseased settings, the use of tissue-specific promoters linked to the differentiation pathway initiated in the recruited stem cell is encompassed in the present invention and could in theory be used to drive the selective expression of therapeutic genes only within a defined biologic context. Stem cells that are recruited to other tissue niches, but do not undergo the same program of differentiation, should not express the therapeutic gene. This approach allows a significant degree of potential control for the selective expression of the therapeutic gene within a defined microenvironment and has been successfully applied to regulate therapeutic gene expression during neovascularization. Potential approaches to such gene modifications are disclosed in WO 2008/150368 and WO 2010/119039.

As used herein, "nucleic acid" shall mean any nucleic acid molecule, including, without limitation, DNA, RNA and hybrids or modified variants thereof. An "exogenous nucleic acid" or "exogenous genetic element" relates to any nucleic acid introduced into the cell, which is not a component of the cells "original" or "natural" genome. Exogenous nucleic acids may be integrated or non-integrated, or relate to stably transfected nucleic acids.

Any given gene delivery method is encompassed by the invention and preferably relates to viral or non-viral vectors, as well as biological or chemical methods of transfection. The methods can yield either stable or transient gene expression in the system used.

Genetically modified viruses have been widely applied for the delivery of genes into stem cells. Preferred viral vectors for genetic modification of the MSCs described herein relate to retroviral vectors, in particular to gamma retroviral vectors. The gamma retrovirus (sometimes referred to as mammalian type C retroviruses) is a sister genus to the lentivirus clade, and is a member of the Orthoretrovirinae subfamily of the retrovirus family. The Murine leukemia virus (MLV or MuLV), the Feline leukemia virus (FeLV), the Xenotropic murine leukemia virus-related virus (XMRV) and the Gibbon ape leukemia virus (GALV) are members of the gamma retrovirus genus. A skilled person is aware of the techniques required for utilization of gamma retroviruses in genetic modification of MSCs. For example, the vectors described by Maetzig et al (Gammaretroviral vectors: biology, technology and application, 2001, Viruses Jun;3(6):677-713) or similar vectors may be employed. For example, the Murine Leukemia Virus (MLV), a simple gammaretrovirus, can be converted into an efficient vehicle of genetic therapeutics in the context of creating gamma retrovirus-modified MSCs and expression of a therapeutic transgene from said MSCs after delivery to a subject.

Adenoviruses may be applied, or RNA viruses such as Lentiviruses, or other retroviruses. Adenoviruses have been used to generate a series of vectors for gene transfer cellular engineering. The initial generation of adenovirus vectors were produced by deleting the EI gene (required for viral replication) generating a vector with a 4kb cloning capacity. An additional deletion of E3 (responsible for host immune response) allowed an 8kb cloning capacity. Further generations have been produced encompassing E2 and/or E4 deletions. Lentiviruses are members of Retroviridae family of viruses (M. Scherr et al., Gene transfer into hematopoietic stem cells using lentiviral vectors. Curr Gene Ther. 2002 Feb; 2(1):45-55). Lentivirus vectors are generated by deletion of the entire viral sequence with the exception of the LTRs and cis acting packaging signals. The resultant vectors have a cloning capacity of about 8 kb. One distinguishing feature of these vectors from retroviral vectors is their ability to transduce dividing and non-dividing cells as well as terminally differentiated cells.

Non-viral methods may also be employed, such as alternative strategies that include conventional plasmid transfer and the application of targeted gene integration through the use of integrase or transposase technologies. These represent approaches for vector transformation that have the advantage of being both efficient, and often site-specific in their integration. Physical methods to introduce vectors into cells are known to a skilled person. One example relates to electroporation, which relies on the use of brief, high voltage electric pulses which create transient pores in the membrane by overcoming its capacitance. One advantage of this method is that it can be utilized for both stable and transient gene expression in most cell types. Alternative methods relate to the use of liposomes or protein transduction domains. Appropriate methods are known to a skilled person and are not intended as limiting embodiments of the present invention.

### FIGURES

The following figures are presented in order to describe particular embodiments of the invention, by demonstrating a practical implementation of the invention, without being limiting to the scope of the invention or the concepts described herein.

### Short description of the figures:

**Fig. 1****:** Preferred expression cassettes of the present invention.
**Fig. 2****:** Titration of retroviral supernatants on HT1080 cells.
**Fig. 3****:** Intracellular flow cytometry analysis of primary human MSCs transduced with viral expression constructs.
**Fig. 4****:** Assessment of transgenic AAT expression by ELISA.
**Fig. 5****:** Inhibition of Neutrophil Elastase by AAT expressed from transduced MSCs.
**Fig. 6****:** Experimental design BLM-induced lung fibrosis model

### Detailed description of the figures:

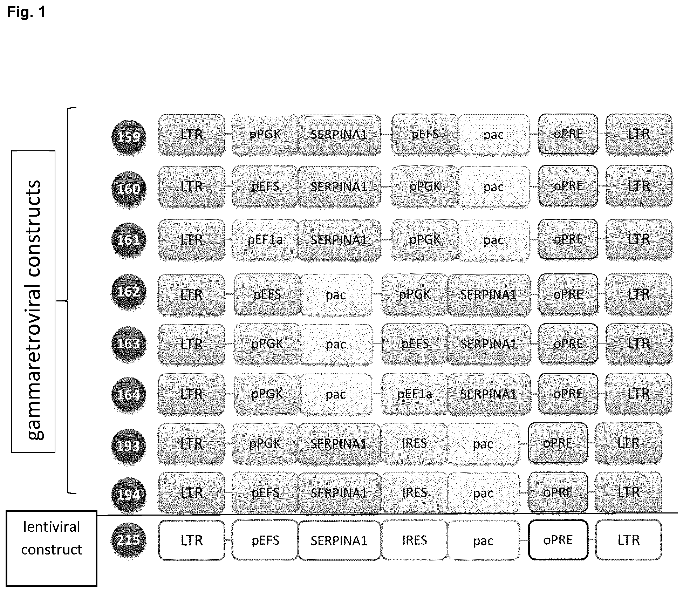

**Fig. 1****:** Preferred expression cassettes of the present invention.
   Schematic representation of the preferred expression cassettes of the present invention. Numbers depicted in the figure represent apceth's internal plasmid denomination and will be used herein for reasons of simplification. The promoters are shown by a lower case p. LTR elements relate to long terminal repeats of the gamma retroviral vector employed. An internal ribosome entry site is abbreviated as IRES. A Posttranscriptional Regulatory Element is abbreviated as oPRE. A puromycin resistance gene (pac) is employed for selection.

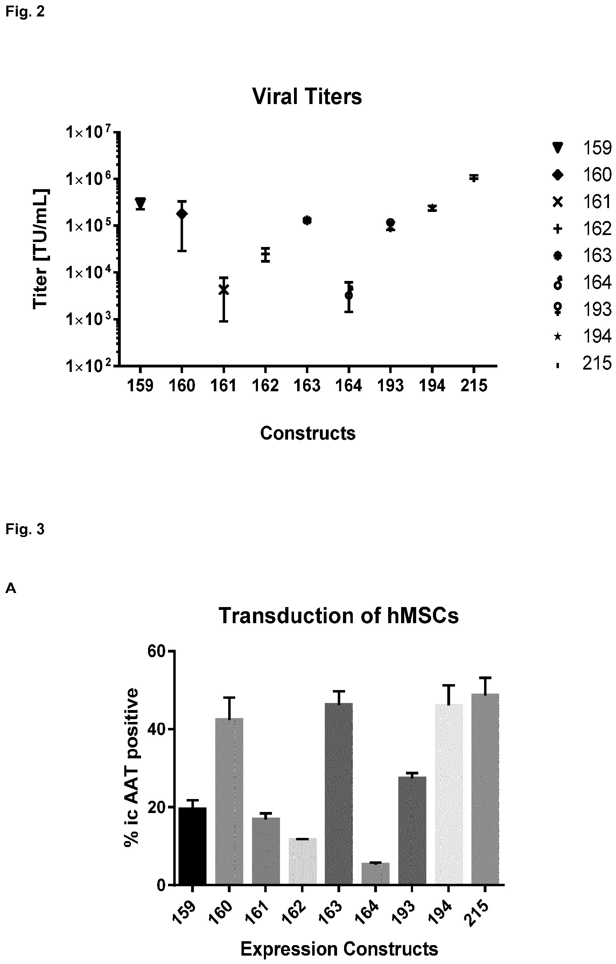

**Fig. 2****:** Titration of retroviral supernatants on HT1080 cells.
   Larger constructs (e.g. 161 and 164 in which the SERPINA1 cDNA is driven by the full length EF1a promoter) yield reduced although sufficient titers as compared to smaller constructs such as ones comprising the EFS promoter (e.g. 159 or 194). In the present experiment, the highest titer is obtained with the lentiviral construct 215.

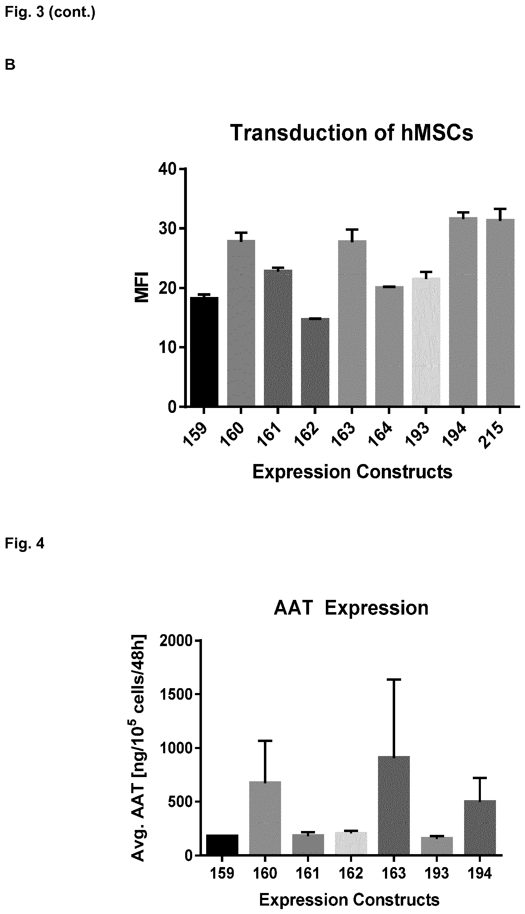

**Fig. 3****:** Intracellular flow cytometry analysis of cells transduced with viral expression constructs.
   Fig. 3a: Intracellular flow cytometry analysis of primary human MSCs transduced with viral expression constructs - %ic AAT positive cells. Fig. 3a depicts percentage of MSCs that stained positive for intracellular AAT after transduction with different gamma-retroviral and lentiviral expression constructs. Fig. 3b: Intracellular flow cytometry analysis of primary human MSCs transduced with viral expression constructs - mean fluorescence intensity (MFI). Fig. 3b depicts mean fluorescence intensity (MFI) values of MSCs transduced with different gamma-retroviral and lentiviral expression constructs. All tested vector constructs are able to transduce primary human MSCs, and transgenic AAT is detected by intracellular flow cytometry in all transduced samples. Differences in transduction efficiency as measured by % ic AAT positive cells are most likely due to different starting titers of the viral supernatants used for transduction. Different expression levels as analyzed by MFI are the result of distinct promoters utilized as well as varied gene cassette constellations.
**Fig. 4****:** Assessment of transgenic AAT expression by ELISA.
   Transgenic AAT expression in primary human MSCs is confirmed in all samples by ELISA. Variances in the amounts expressed are due to distinct promoters utilized in the vectors as well as varied gene cassette constellations.

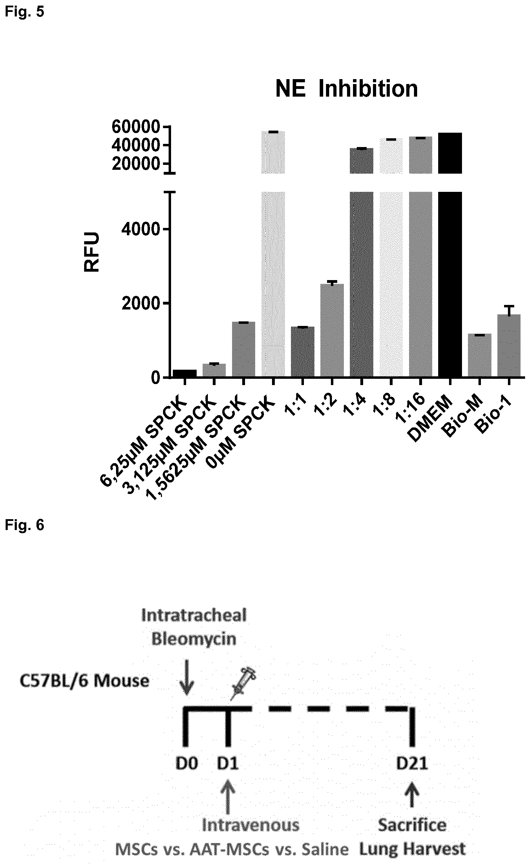

**Fig. 5****:** Inhibition of Neutrophil Elastase by AAT expressed from transduced MSCs.
   AAT expressed from transduced primary human MSCs is functional and inhibits Neutrophil Elastase at levels comparable to medium containing 10% serum or ∼1.5µM SPCK.
**Fig. 6****:** Experimental design BLM-induced lung fibrosis model (adapted from Tashiro et al., 2015).

### EXAMPLES

The invention is further described by the following examples. These are not intended to limit the scope of the invention. The experimental examples relate to the development of technology that enables Alpha-1 antitrypsin (AAT) expression from genetically modified MSCs. The examples further relate to therapeutic trials encompassing the treatment of conditions of the lung.

In preferred embodiments the examples relate to the preclinical development of a novel gene therapy product that combines the anti-inflammatory effect of Alpha-1 antitrypsin (AAT) with the immunomodulatory properties of primary human mesenchymal stem cells (MSCs) for the treatment of inflammatory lung diseases.

### 1. Design and cloning of retroviral vector constructs:

The transgene expression cassettes are constructed using standard cloning techniques as described in Julia Lodge, Peter Lund, Steve Minchin (2007) Gene Cloning, New York: Tylor and Francis Group. The gene expressed by these constructs is the human SERPINA1 cDNA {Homo sapiens serpin peptidase inhibitor, clade A (alpha-1 antiproteinase, antitrypsin), member 1 (SERPINA1), transcript variant 1, mRNA; NCBI Reference Sequence: NM_000295.4, encoding Alpha-1 antitrypsin (AAT)}. A codon optimized cDNA as described above according to SEQ ID NO 2 has also been assessed.

The SERPINA1 gene as described herein is expressed by activation of different constitutive promoters, such as the human EEF1A1 eukaryotic translation elongation factor 1 alpha 1 promoter (pEF1a), the short form of the human EEF1A1 eukaryotic translation elongation factor 1 alpha 1 promoter (pEFS), or the human phosphoglycerate kinase promoter (pPGK). The promoters may also be inducible promoters like Tie2, RANTES or the HSP70 promoter.

The gene may or may not be fused with tag-sequences (e.g. marker proteins/peptides such as the hemagglutinin tag or the HIS tag) to allow for easy detection of expression later on (Hinrik Garoff, 1985, Annual Review of Cell Biology, Vol. 1: 403-445).

The expression cassette may or may not include a second transgene cassette consisting of a selectable marker gene, such as a cell surface marker or a resistance gene (for example the pac gene to confer puromycin resistance) to allow for enrichment of genetically modified cells later in the process (David P. Clark, Nanette J. Pazdernik, 2009, Biotechnology: Applying the Genetic Revolution, London: Elsevier). The gene is either driven by a separate promoter or located 3' of an IRES sequence within the SERPINA1 expression cassette.

To assess potential positional effects, SERPINA1 and pac cassettes are cloned in different constellations (SERPINA1 cassette 5' of pac cassette and vice versa). Refer to Figure 1.

The expression cassettes disclosed in Figure 1 are then inserted into a suitable vector system, e.g. a gamma-retroviral (e.g. pSERS11, EP2019134A1) or lentiviral backbone (e.g. United States Patent 8,846,385,) by standard cloning techniques.

In the gamma-retroviral constructs, there is an oPRE sequence 3' of the expression cassette(s).

The retroviral backbone contains long terminal repeats (LTRs) which are located at the 5'- and 3'-end of the cassette. The 5'-LTR contains an SV40 enhancer, RSV promoter, an SFFVp R and U5 region. The 3'-LTR contains an SFFVp U3 region that has a deletion and thus renders the vector self-inactivating (SIN), an SFFV R and U5 region as well as a PolyA signal.

In the lentiviral construct, there also is an oPRE sequence 3' of the expression cassette(s). The lentiviral backbone contains long terminal repeats (LTRs) which are located at the 5'- and 3'-end of the cassette. The 5'-LTR contains a CMV promoter and an HIV-1 R and U5 region. The 3'-LTR contains an HIV-1 U3 region that has a deletion and thus renders the vector self-inactivating (SIN), an HIV-1 R and U5 region as well as a PolyA signal.

### 2. Titration of retroviral supernatants:

Viral particles encoding the designated vectors are generated by transient transfection of 293T cells (Soneoka et al., Nucleic Acids Research, 1995). In order to determine viral titers, HT1080 fibrosacroma cells are seeded in 12-well plates on day 1, viral supernatants at different dilutions are added to cells on day 2, and three control wells are used to determine cell numbers per well. Three days after transduction, transduction efficiency is analyzed by an intracellular flow cytometry assay detecting AAT protein. To enhance the detection of the protein, cells are treated with GolgiPlug Protein Transport Inhibitor (BD, 555029) for 16 to 24 hours before the staining to prevent the secretion of cytosolic proteins. Cells are permeabilized using BD Cytofix/Cytoperm Fixation and Permeabilization Solution (BD, 554722) according to manufacturer's instructions, and AAT-expressing cells are stained with a FITC-conjugated Anti-alpha 1 Antitrypsin antibody (abcam, ab19170; 1µL antibody per 100µL staining reaction and up to 1x10⁶ cells, incubation for 20-30 minutes at 4°C in the dark). Cells are analyzed on a Beckman Coulter FC500 flow cytometer. Only values <25% ic AAT positive cells are included in titer calculations.

Refer Figure 2 for results.

### 3. Preparation of human mesenchymal stem cells (MSCs):

Human MSCs are isolated from bone marrow by plastic adherence and are cultured in growth medium e.g. FBS containing DMEM as described by Pittinger, M.F. (2008) Mesenchymal stem cells from adult bone marrow, In D.J. Prockop, D.G. Phinney, B.A. Bunnell, Methods in Molecular Biology 449, Mesenchymal stem cells, Totowa: Humana Press.

### 4. Genetic modification of MSCs:

The transduction of primary MSCs is performed with modifications as described by Murray et al., 1999 Human Gene Therapy. 10(11): 1743-1752 and Davis et al., 2004 Biophysical Journal Volume 86 1234-1242. In detail: 6-well or 12-well cell culture plates (e.g. Corning) are coated with Poly-L-Lysine (PLL) (e.g. Sigma-Aldrich, P4707-50mL); the PLL solution (0.01%) is diluted to a final concentration of 0.001% with PBS. 1 to 2mL of diluted PLL are used for each well. The plate is incubated for at least 2 hours at room temperature. After incubation, the plates are washed once with PBS. (Diluted) viral supernatant is added to each PLL-coated well in a final volume of 0.8 to 2mL. The loaded plate is centrifuged for 2000xg for 30min at 4°C. The supernatant is then discarded and 1x10⁵ MSCs are seeded in one well of a 6-well plate in a volume of 2mL or 4x10⁴ cells are seeded in a well of a 12-well plate in a volume of 1mL. The plates are incubated at 37°C with 5% CO₂ for further use.

### 5. Assessment of transgenic AAT expression by intracellular flow cytometry:

To assess transduction efficiency and AAT expression in primary human MSCs, cells are prepared and transduced as described above with multiplicities of infection (MOIs) of 0.25 to 10. Transduced cells are selected with puromycin (Sigma Aldrich, P9620-10mL, [10 mg/mL], final concentration: 1-5µg/mL) for 5 to 8 days. Selected cells are analyzed by intracellular flow cytometry as described above.

All tested vector constructs are able to transduce primary human MSCs, and transgenic AAT is detected by intracellular flow cytometry in all transduced samples. Differences in transduction efficiency as measured by % ic AAT positive cells are most likely due to different starting titers of the viral supernatants used for transduction, such that any of the provided cell population upon further isolation and culturing may provide suitable AAT expression. Different expression levels as analyzed by MFI are the result of distinct promoters utilized as well as varied gene cassette constellations.

Refer to Figure 3 for results.

### 6. Assessment of transgenic AAT expression by ELISA:

Human MSCs are transduced with the indicated retroviral constructs expressing AAT and the pac gene. The cells are selected with puromycin as described above and 1x10⁵ cells are seeded onto 6- or 12-well plates. Supernatants (1-2mL) are collected after 48 hours and analyzed by ELISA (alpha 1 Antitrypsin (SERPINA1) Human ELISA Kit, abcam, ab108799) according to manufacturer's instructions. Data generated are normalized to 1x10⁵ cells and vector copy number (VCN).

Transgenic AAT expression in primary human MSCs is confirmed in all samples by ELISA. Variances in the amounts expressed are due to distinct promoters utilized in the vectors as well as varied gene cassette constellations, such that each of the examples provided enables sufficient AAT expression at the protein level to obtain a desired effect.

Refer to Figure 4 for results.

### 7. Inhibition of Neutrophil Elastase by AAT expressed from transduced MSCs:

Human MSCs are transduced with the indicated retroviral constructs expressing AAT and the pac gene. The cells are selected with puromycin and selected cells are seeded onto 6- or 12-well plates in DMEM without serum. Supernatants (1-2mL) are collected after 48 hours and analyzed by a Neutrophil Elastase Inhibitor Screening Kit (abcam, ab118971) according to manufacturer's instructions. Supernatant of transduced MSCs is analyzed in different dilutions ranging from non-diluted (1:1) to 1:16 in DMEM without serum. SPCK in different concentrations and medium containing serum (Bio-M and Bio-1) are included as positive controls, DMEM is used as a negative control.

Inhibition of neutrophil elastase is a functional assay for detecting AAT activity in in vitro. The constructs provided herein show effective inhibition of neutrophil elastase thereby indicating expression of functional AAT from the modified MSCs of the examples.

Refer to Figure 5 for results.

### 8. Assessment of immunomodulatory effect of AAT expressed from MSCs on Monocytes:

Peripheral blood mononuclear cells (PBMCs) are isolated from human blood using ficoll density gradient centrifugation as described by Ivan J. Fuss, Marjorie E. Kanof, Phillip D. Smith, Heddy Zola, 2009 Curr. Protoc. Immunol. 85: 7.1.1-7.1.8. To assess the immunomodulatory effect of AAT-MSCs *in vitro,* an assay of LPS-induced human monocyte activation is performed as described in Janciauskiene et al., Biochemical and Biophysical Research Communications, 2004. In brief, monocytes are stimulated with lipopolysaccharide (LPS) and the effect of AAT expressed from MSCs on the secretion of proinflammatory cytokines such as TNFalpha and IL-1beta as well as on the expression of anti-inflammatory cytokines such as IL-10 from human monocytes is assessed in supernatants by ELISA.

When human primary monocytes are cultured in the presence of AAT secreted from genetically modified MSCs (supernatants from MSCs transduced to express AAT), the expression of proinflammatory cytokines (TNFalpha, IL-1beta) in supernatants harvested from monocyte cultures is markedly decreased, whereas there is an increase in the levels of anti-inflammatory cytokines such as IL-10.

### 9. Assessment of immunomodulatory effect of AAT-MSC administration in animal models:

Cells used in *in vivo* experiments are prepared and genetically modified as described above. Transduced cells are selected and expanded further before either cryopreservation or harvest for administration. The cells are either thawed, washed with and re-suspended in PBS or any other suitable buffer prior to injection, or detached from the culture flasks, washed with and re-suspended in PBS or any other suitable buffer, and then injected.

### 10. Bleomycin (BLM)-induced pulmonary fibrosis:

To test the immunomodulatory and anti-fibrotic effect of AAT-MSCs *in vivo,* a mouse model of bleomycin-induced pulmonary fibrosis is performed as described in Tashiro et al., Translational Science 2015.

In brief, BLM lung fibrosis is induced in C57BL/6 mice. After the administration of anesthesia, BLM sulfate (Sigma-Aldrich) dissolved in 50µL of sterile saline at 2.5U per kg of body weight is administered by direct intratracheal instillation via intubation. 24 to 72 hours after BLM administration, each animal receives 200µL either PBS (control), 1x10⁶ non-transduced MSCs, or 1x10⁶ AAT-transduced MSCs in 200µL PBS by tail vain injection or intratracheal administration. Serum AAT-levels are monitored by retro-orbital blood collection every second day and subsequent detection of AAT protein by ELISA.

Mice are killed at 14-21 days after BLM administration.

Left lung lobes are harvested from mice for protein and messenger RNA (mRNA) analysis. For morphometry and histology studies, right lung lobes are fixed by immersion in 10% neutral-buffered formalin for 24 hours and then transferred to PBS at 4°C. Samples are paraffin embedded, and sections are obtained for hematoxylin-eosin and Masson trichrome staining. Pulmonary fibrosis is assessed using the semiquantitative Ashcroft method on Masson trichrome-stained slides (Ashcroft et al., Journal of Clinical Pathology 1988).

Refer Figure 6 for an overview of the experimental design.

At the time point of 21-day sacrifice, BLM mice without MSC treatment demonstrate lung fibrosis by Ashcroft score, whereas mice treated with either MSCs or AAT-MSCs showed decreased fibrosis. The decrease was more marked in the group receiving MSCs expressing AAT.

### 11. Cyclophosphamide-accelerated Type 1 Diabetes:

To assess the effect of AAT-MSCs on the development of diabetes *in vivo,* a mouse model of cyclophosphamide-accelerated type 1 diabetes is performed (adapted from Brode et al., The Journal of Immunology 2006).

NOD mice are obtained, where the incidence of diabetes in female mice is 75% by 40 weeks of age. To accelerate and synchronize diabetes, female 8-week-old NOD mice are treated with a single i.p. injection of cyclophosphamide (CY) (200 mg/kg body weight in 0.9% normal saline). Mice are then randomly divided into treatment and control groups. One to five days after cyclophosphamide treatment, each animal receives 200µL either PBS (control), 1x10⁶ non-transduced MSCs, or 1x10⁶ AAT-transduced MSCs in 200µL PBS by tail vain or intraperitoneal injection. Mice are monitored weekly for hyperglycemia until they become diabetic, as defined by two consecutive (>24 hr apart) nonfasting blood glucose levels >240 mg/dl.

All control mice receiving CY and PBS developed diabetes within 30 days, whereas the onset of diabetes in mice treated with either MSCs or AAT-MSCs was delayed. Interestingly, in mice treated with AAT-MSCs, the delay in development of diabetes was increased by 2 weeks compared to non-modified MSCs.

### 12. MSU/C16.0-induced gouty arthritis:

To assess the anti-inflammatory effect of AAT-MSCs on gouty arthritis *in vivo,* a mouse model of MSU/C16.0-induced gouty arthritis is performed (adapted from Joosten et al., Annals of the Rheumatic Diseases 2015).

Male C57BI/6 mice are obtained from Jackson Laboratories (Bar Harbor, Maine, USA) and are used at 10-12 weeks. One to three days prior to induction of gouty arthritis, each animal receives 200µL either PBS (control), 1x10⁶ non-transduced MSCs, or 1x10⁶ AAT-transduced MSCs in 200µL PBS by intraperitoneal administration. Joint inflammation is induced by intra-articular injection of 300µg MSU crystals mixed with 200µM C16.0/bovine serum albumin (BSA) in 10µL PBS into the right knee joint of naive mice. Four hours after intra-articular injection, macroscopic joint swelling is determined. Synovial tissue is isolated and either cultured for 2 hours in tissue culture medium at 37°C or transferred directly into 200µL Triton×100 (0.5% in PBS). In addition, knee joints are removed for histology.

Treatment with either non-modified or AAT-MSCs suppressed MSCU/C16.0 induced joint inflammation; however, inflammation was more markedly decreased when mice were treated with AAT-MSCs.

### 13. Effect of AAT-MSCs on GvHD prevention in an MHC matched, minor antigen disparate murine transplant model:

For the assessment of a possible anti-GvHD effect of AAT-MSCs, a murine transplant model (MHC matched, minor antigen disparate) is performed (adapted from Marcondes et al., Blood 2011).

C57/BL6J mice (H-2^{b}; The Jackson Laboratory), 10-14 weeks old with average body weight of 28g, receive single-dose total body irradiation with 1000cGy followed by intra-tail vein injection of T-cell depleted bone marrow (5x10⁶ cells), and CD8+ splenic lymphocytes (0.2x10⁶cells) from C3H.SW-H2b/SnJ donors (H-2bc; The Jackson Laboratory). Mice are randomly divided into treatment and control groups. Mice in the experimental group are given either 1x10⁶ non-transduced MSCs or 1x10⁶ AAT-transduced MSCs in 200µL PBS by intraperitoneal or intra-tail vein injection before irradiation and donor cell infusion. Mice in the control group are injected, also intraperitoneally or intravenously, with 200µL PBS. GvHD is assessed by a standard scoring system (Cooke et al., Blood 1996): Body weights were obtained and recorded on day 0 and weekly thereafter. A weekly clinical index is generated by summation of 5 criteria scores: percentage of weight change, posture (hunching), activity, fur texture, and skin integrity (maximum score = 10). Blood samples are collected sequentially for cytokine assays.

Treatment with either non-modified MSCs or AAT-MSCs results in attenuation or prevention of GvHD and superior survival compared to control mice. Interestingly, the beneficial effect was more prominent in AAT-MSCs compared to native MSCs.

## Claims

1. Genetically modified mesenchymal stem cell for use as a medicament in the treatment of medical conditions associated with inflammation and/or an unwanted immune response in subjects without an alpha1-antitrypsin (AAT) deficiency, wherein said stem cells comprise an exogenous nucleic acid comprising (i) an Alpha-1 antitrypsin (AAT) encoding region operably linked to (ii) a promoter or promoter/enhancer combination.

2. The genetically modified cell for use as a medicament according to claim 1, wherein the exogenous nucleic acid consists of or comprises a viral vector, more preferably a retroviral vector.

3. The genetically modified cell for use as a medicament according to the preceding claim, wherein the viral vector is a lentiviral vector.

4. The genetically modified cell for use as a medicament according to any one of the preceding claims, wherein the promoter or promoter/enhancer combination is a constitutive promoter.

5. The genetically modified cell for use as a medicament according to the preceding claim, wherein the constitutive promoter is the EFS, PGK, or EF1alpha promoter.

6. The genetically modified cell for use as a medicament according to any one of claims 1-3, wherein said promoter or promoter/enhancer combination is an inducible promoter.

7. The genetically modified cell for use as a medicament according to the preceding claim, wherein the promoter is inducible upon differentiation of said cell post-administration or the promoter is an inflammation-specific promoter.

8. The genetically modified cell for use as a medicament according to any one of the preceding claims, wherein said cells are administered by introducing a therapeutically effective number of cells into the bloodstream of a patient, preferably via intravenous injection.

9. The genetically modified cell for use as a medicament according to the preceding claim, wherein the medical condition associated with inflammation and/or an unwanted immune response is a lung disease, preferably an inflammatory disease of the lung.

10. The genetically modified cell for use as a medicament in the treatment of a lung disease according to claim 9, wherein said lung disease is a respiratory disease.

11. The genetically modified cell for use as a medicament in the treatment of a lung disease according to any one of claims 9 or 10, wherein the lung disease is acute lung injury, chronic obstructive pulmonary disease (COPD) including chronic bronchitis, emphysema, bronchiectasis and bronchiolitis, acute respiratory distress syndrome, asthma, sarcoidosis, hypersensitivity pneumonitis and/or pulmonary fibrosis.

12. The genetically modified cell for use as a medicament in the treatment of a lung disease according to any one of the preceding claims, wherein said cells are administered by introducing a therapeutically effective number of cells to the lung of a patient by inhalation, optionally in combination with introduction of said cells into the bloodstream of a patient.

13. The genetically modified cell for use as a medicament according to any one of the preceding claims, wherein the medical condition associated with inflammation and/or an unwanted immune response is gout, chronic fibrosis or an autoimmune disease, wherein the inflammatory and/or chronic fibrotic disease is preferably of the kidney, liver and/or colon of a subject.

14. The genetically modified cell for use as a medicament according to any one of the preceding claims, wherein the medical condition associated with inflammation and/or an unwanted immune response is an inflammatory disease selected from vasculitis, nephritis, inflammatory bowel disease, rheumatoid arthritis and/or Graft versus Host disease.

15. The genetically modified cell according to claim 13 for use as a medicament, wherein the autoimmune disease is diabetes Type 1.

## Patentansprüche

1. Genetisch modifizierte mesenchymale Stammzellen zur Verwendung als ein Medikament in der Behandlung von medizinischen Zuständen, die mit Entzündung und/oder einer unerwünschten Immunantwort assoziiert sind, in Subjekten ohne einen Alpha-1-Antitrypsin(AAT)-Mangel wobei die Stammzellen eine exogene Nukleinsäure umfassen, die (i) eine Alpha-1-Antitrypsin (AAT) kodierende Region umfasst, die (ii) mit einem Promoter oder einer Promoter-Verstärker-Kombination wirkverbunden ist.

2. Genetisch modifizierte Zelle zur Verwendung als ein Medikament nach Anspruch 1, wobei die exogene Nukleinsäure einen viralen Vektor, vorzugsweise einen retroviralen Vektor umfasst oder aus diesem besteht.

3. Genetisch modifizierte Zelle zur Verwendung als ein Medikament nach dem vorstehenden Anspruch, wobei der virale Vektor ein lentiviraler Vektor ist.

4. Genetisch modifizierte Zelle zur Verwendung als ein Medikament nach einem der vorstehenden Ansprüche, wobei der Promoter oder die Promoter-Verstärker-Kombination ein konstitutiver Promotor ist.

5. Genetisch modifizierte Zelle zur Verwendung als ein Medikament nach dem vorstehenden Anspruch, wobei der konstitutive Promoter der EFS-, PGK- oder EF1alpha-Promoter ist.

6. Genetisch modifizierte Zelle zur Verwendung als ein Medikament nach einem der vorstehenden Ansprüche 1-3, wobei der Promoter oder die Promoter-Verstärker-Kombination ein induzierbarer Promotor ist.

7. Genetisch modifizierte Zelle zur Verwendung als ein Medikament nach dem vorstehenden Anspruch, wobei der Promoter bei Differenzierung der Zelle nach der Verabreichung induzierbar ist oder der Promoter ein entzündungsspezifischer Promoter ist.

8. Genetisch modifizierte Zelle zur Verwendung als ein Medikament nach einem der vorstehenden Ansprüche, wobei die Zellen durch Einführen einer therapeutisch wirksamen Anzahl von Zellen in den Blutkreislauf eines Patienten, vorzugsweise durch intravenöse Injektion, verabreicht werden.

9. Genetisch modifizierte Zelle zur Verwendung als ein Medikament nach dem vorstehenden Anspruch, wobei der medizinische Zustand, der mit Entzündung und/oder einer unerwünschten Immunantwort assoziiert ist, eine Lungenerkrankung, vorzugsweise eine entzündliche Lungenerkrankung, ist.

10. Genetisch modifizierte Zelle zur Verwendung als ein Medikament in der Behandlung einer Lungenerkrankung nach Anspruch 9, wobei die Lungenerkrankung eine Atemwegserkrankung ist.

11. Genetisch modifizierte Zelle zur Verwendung als ein Medikament in der Behandlung einer Lungenerkrankung nach einem der Ansprüche 9 oder 10, wobei die Lungenerkrankung akute Lungenerkrankung, chronisch obstruktive Lungenerkrankung (COPD) einschließlich chronischer Bronchitis, Emphysem, Bronchiektasie, Bronchiolitis, akutes Atemnotsyndrom, Asthma, Sarkoidose, Hypersensitivitäts-Pneumonitis und/oder Lungenfibrose ist.

12. Genetisch modifizierte Zelle zur Verwendung als ein Medikament in der Behandlung einer Lungenerkrankung nach einem der vorstehenden Ansprüche, wobei die Zellen durch Einführen einer therapeutisch wirksamen Anzahl von Zellen in die Lunge eines Patienten durch Inhalation verabreicht werden, gegebenenfalls in Kombination mit dem Einführen der Zellen in den Blutkreislauf eines Patienten.

13. Genetisch modifizierte Zelle zur Verwendung als ein Medikament nach einem der vorstehenden Ansprüche, wobei der medizinische Zustand, der mit Entzündung und/oder einer unerwünschten Immunantwort assoziiert ist, Gicht, chronische Fibrose oder eine Autoimmunerkrankung ist, wobei die entzündliche und/oder chronische fibrotische Erkrankung vorzugsweise eine der Niere, der Leber und/oder des Dickdarms eines Subjekts ist.

14. Genetisch modifizierte Zelle zur Verwendung als ein Medikament nach einem der vorstehenden Ansprüche, wobei der medizinische Zustand, der mit Entzündung und/oder einer unerwünschten Immunantwort assoziiert ist, eine entzündliche Erkrankung ist, die aus Vasculitis, Nephritis, entzündlicher Darmerkrankung, rheumatoider Arthritis und/der Graft-versus-Host-Erkrankung ausgewählt ist.

15. Genetisch modifizierte Zelle nach Anspruch 13 zu Verwendung als ein Medikament, wobei die Autoimmunerkrankung Diabetes Typ 1 ist.

## Revendications

1. Cellule souche mésenchymateuse génétiquement modifiée à utiliser en tant que médicament dans le traitement de pathologies médicales associées à une inflammation et/ou à une réponse immunitaire non désirée chez des patients sans carence en alpha-1-antitrypsine (AAT), dans laquelle lesdites cellules souches comprennent un acide nucléique exogène comprenant (i) une région encodant l'alpha-1-antitrypsine (AAT) liée de manière fonctionnelle à (ii) un promoteur ou une combinaison promoteur/amplificateur.

2. Cellule génétiquement modifiée à utiliser en tant que médicament selon la revendication 1, dans laquelle l'acide nucléique exogène se compose de ou comprend un vecteur viral, de préférence un vecteur rétroviral.

3. Cellule génétiquement modifiée à utiliser en tant que médicament selon la revendication précédente, dans laquelle le vecteur viral est un vecteur lentiviral.

4. Cellule génétiquement modifiée à utiliser en tant que médicament selon l'une quelconque des revendications précédentes, dans laquelle le promoteur ou la combinaison promoteur/amplificateur est un promoteur constitutif.

5. Cellule génétiquement modifiée à utiliser en tant que médicament selon la revendication précédente, dans laquelle le promoteur constitutif est le promoteur EFS, PGK OU EF1alpha.

6. Cellule génétiquement modifiée à utiliser en tant que médicament selon l'une quelconque des revendications 1 à 3, dans laquelle ledit promoteur ou ladite combinaison promoteur/amplificateur est un promoteur inductible.

7. Cellule génétiquement modifiée à utiliser en tant que médicament selon la revendication précédente, dans laquelle le promoteur est inductible en cas de différenciation de ladite post-administration cellulaire ou le promoteur est un promoteur spécifique à l'inflammation.

8. Cellule génétiquement modifiée à utiliser en tant que médicament selon l'une quelconque des revendications précédentes, dans laquelle lesdites cellules sont administrées en introduisant un nombre thérapeutiquement efficace de cellules dans la circulation sanguine d'un patient, de préférence par injection intraveineuse.

9. Cellule génétiquement modifiée à utiliser en tant que médicament selon la revendication précédente, dans laquelle la pathologie médicale associée à une inflammation et/ou à une réponse immunitaire non désirée est une maladie pulmonaire, de préférence une maladie inflammatoire du poumon.

10. Cellule génétiquement modifiée à utiliser en tant que médicament dans le traitement d'une maladie pulmonaire selon la revendication 9, dans laquelle ladite maladie pulmonaire est une maladie respiratoire.

11. Cellule génétiquement modifiée à utiliser en tant que médicament dans le traitement d'une maladie pulmonaire selon l'une quelconque des revendications 9 ou 10, dans laquelle la maladie pulmonaire est une maladie pulmonaire aiguë, une bronchopneumopathie chronique obstructive (BPCO) comprenant une bronchite chronique, un emphysème, une bronchectasie et une bronchiolite, un syndrome de détresse respiratoire aiguë, de l'asthme, une sarcoïdose, une alvéolite allergique extrinsèque et/ou une fibrose pulmonaire.

12. Cellule génétiquement modifiée à utiliser en tant que médicament dans le traitement d'une maladie pulmonaire selon l'une quelconque des revendications précédentes, dans laquelle lesdites cellules sont administrées en introduisant un nombre thérapeutiquement efficace de cellules dans le poumon d'un patient par inhalation, éventuellement en combinaison avec l'introduction desdites cellules dans la circulation sanguine d'un patient.

13. Cellule génétiquement modifiée à utiliser en tant que médicament selon l'une quelconque des revendications précédentes, dans laquelle la pathologie médicale associée à une inflammation et/ou à une réponse immunitaire non désirée est la goutte, une fibrose chronique ou une maladie auto-immune, dans laquelle la maladie inflammatoire et/ou fibrotique chronique concerne de préférence le rein, le foie et/ou le côlon d'un patient.

14. Cellule génétiquement modifiée à utiliser en tant que médicament selon l'une quelconque des revendications précédentes, dans laquelle la pathologie médicale associée à une inflammation et/ou à une réponse immunitaire non désirée est une maladie inflammatoire choisie parmi la vascularite, la néphrite, l'affection abdominale inflammatoire, l'arthrite rhumatoïde et/ou une réaction de greffe contre hôte.

15. Cellule génétiquement modifiée selon la revendication 13 à utiliser en tant que médicament, dans laquelle la maladie auto-immune est le diabète de type 1.


### SEQUENCE LISTING

<110> apceth GmbH & Co. KG
<120> GENETICALLY MODIFIED MESENCHYMAL STEM CELLS EXPRESSING ALPHA-1 ANTITRYPSIN (AAT)
<130> XII 2275/14WO
<150> EP 15150454.5
   <151> 2015-01-08
<150> EP 15191934.7
   <151> 2015-10-28
<160> 3
<170> PatentIn version 3.5
<210> 1
   <211> 1257
   <212> DNA
   <213> Homo sapiens
<400> 1
   atgccgtctt ctgtctcgtg gggcatcctc ctgctggcag gcctgtgctg cctggtccct      60
   gtctccctgg ctgaggatcc ccagggagat gctgcccaga agacagatac atcccaccat     120
   gatcaggatc acccaacctt caacaagatc acccccaacc tggctgagtt cgccttcagc     180
   ctataccgcc agctggcaca ccagtccaac agcaccaata tcttcttctc cccagtgagc     240
   atcgctacag cctttgcaat gctctccctg gggaccaagg ctgacactca cgatgaaatc     300
   ctggagggcc tgaatttcaa cctcacggag attccggagg ctcagatcca tgaaggcttc     360
   caggaactcc tccgtaccct caaccagcca gacagccagc tccagctgac caccggcaat     420
   ggcctgttcc tcagcgaggg cctgaagcta gtggataagt ttttggagga tgttaaaaag      480
   ttgtaccact cagaagcctt cactgtcaac ttcggggaca ccgaagaggc caagaaacag     540
   atcaacgatt acgtggagaa gggtactcaa gggaaaattg tggatttggt caaggagctt     600
   gacagagaca cagtttttgc tctggtgaat tacatcttct ttaaaggcaa atgggagaga     660
   ccctttgaag tcaaggacac cgaggaagag gacttccacg tggaccaggt gaccaccgtg     720
   aaggtgccta tgatgaagcg tttaggcatg tttaacatcc agcactgtaa gaagctgtcc     780
   agctgggtgc tgctgatgaa atacctgggc aatgccaccg ccatcttctt cctgcctgat     840
   gaggggaaac tacagcacct ggaaaatgaa ctcacccacg atatcatcac caagttcctg     900
   gaaaatgaag acagaaggtc tgccagctta catttaccca aactgtccat tactggaacc     960
   tatgatctga agagcgtcct gggtcaactg ggcatcacta aggtcttcag caatggggct    1020
   gacctctccg gggtcacaga ggaggcaccc ctgaagctct ccaaggccgt gcataaggct    1080
   gtgctgacca tcgacgagaa agggactgaa gctgctgggg ccatgttttt agaggccata    1140
   cccatgtcta tcccccccga ggtcaagttc aacaaaccct ttgtcttctt aatgattgaa     1200
   caaaatacca agtctcccct cttcatggga aaagtggtga atcccaccca aaaataa       1257
<210> 2
   <211> 1254
   <212> DNA
   <213> Homo sapiens
<400> 2
   atgcccagca gcgtgtcctg gggaattctg ctgctggccg gcctgtgttg tctggtgcct        60
   gtgtctctgg ccgaggaccc tcagggggat gccgcccaga aaaccgatac cagccaccac       120
   gaccaggacc accccacctt caacaagatc acccccaacc tggccgagtt cgccttcagc       180
   ctgtacagac agctggccca ccagagcaac agcaccaaca tctttttcag ccccgtgtct       240
   atcgccaccg ccttcgccat gctgagcctg ggcacaaagg ccgacaccca cgacgagatc       300
   ctggaaggcc tgaacttcaa cctgaccgag atccccgagg cccagatcca cgagggcttc       360
   caggaactgc tgcggaccct gaaccagccc gatagccagc tgcagctgac aaccggcaac       420
   ggcctgtttc tgagcgaggg actgaagctg gtggacaagt ttctggaaga tgtgaagaag       480
   ctgtatcaca gcgaggcctt caccgtgaac ttcggcgaca ccgaggaagc caagaagcag       540
   atcaacgact acgtggaaaa gggcacccag ggcaagatcg tggacctcgt gaaagagctg       600
   gaccgggaca ccgtgttcgc cctcgtgaac tacatcttct tcaagggcaa gtgggagcgg       660
   cccttcgaag tgaaggacac agaggaagag gactttcacg tggaccaagt gaccaccgtg       720
   aaggtgccca tgatgaagag actgggcatg ttcaacatcc agcactgcaa gaaactgagc       780
   agctgggtgc tgctgatgaa gtacctgggc aacgctaccg ccatattctt tctgcccgac       840
   gagggcaagc tgcagcacct ggaaaacgag ctgacccacg acatcatcac caaatttctg       900
   gaaaatgagg accggcggag cgccagcctg catctgccta agctgtctat caccggcacc       960
   tacgacctga agtccgtgct gggacagctg ggcatcacca aggtgttcag caacggcgcc      1020
   gatctgagcg gcgtgacaga agaggcccct ctgaagctgt ccaaggccgt gcacaaagcc      1080
   gtgctgacca tcgacgagaa gggcaccgaa gccgctggcg ccatgtttct ggaagccatc      1140
   cccatgagca tcccccctga agtgaagttc aacaagccct tcgtgttcct gatgatcgag      1200
   cagaacacca agagccccct gttcatgggc aaggtcgtga accccaccca gaaa            1254
<210> 3
   <211> 418
   <212> PRT
   <213> Homo sapiens
<400> 3